# FASCICULE DE BREVET EUROPEEN

(11) **EP 4 463 113 B1**
(45) Date de publication et mention de la délivrance du brevet: **31.12.2025**
(21) Numéro de dépôt: 23702641.4
(22) Date de dépôt: 12.01.2023
(51) Int. Cl.: A61F 9/00

(54) **DISPOSITIF DE DELIVRANCE D'UNE GOUTTE DE COLLYRE**
AUGENTROPFENAUSGABEVORRICHTUNG
EYE DROP DISPENSING DEVICE

(30) Priorité: 14.01.2022 FR 2200304
(43) Date de publication de la demande: 20.11.2024
(73) Titulaire: Akrivision Technologies, 67370 Truchtersheim (FR)
(72) Inventeur: DAULL, Philippe, 91450 Soisy Sur Seine (FR); ROY, Pierre, 75019 PARIS (FR)
(74) Mandataire: Tranvouez, Edern Morgan
(86) Numéro de dépôt international: PCT/FR2023/000004
(87) Numéro de publication internationale: WO 2023/135376

(56) Documents cités:
- EP-A1- 1 724 203
- WO-A1-98/55059
- US-A1- 2005 288 640

## Description

### DOMAINE TECHNIQUE DE L'INVENTION

L'invention concerne le domaine des dispositifs de délivrance d'une goutte de collyre, comportant ou non un ou plusieurs principes actifs.

### ETAT DE LA TECHNIQUE ANTERIEURE

Actuellement, les dispositifs de délivrance d'une goutte de collyre sont conformés dans leurs grandes majorités de sorte à ce que l'utilisateur patient doit pencher la tête en arrière et basculer le dispositif de délivrance d'une goutte de collyre pour que l'extrémité de distribution de ce dernier soit sensiblement au-dessus de l'œil ciblé du patient. Une fois dans cette position, le patient appuie généralement sur le corps du flacon associé au dispositif de délivrance afin de faire tomber une goutte de collyre vers l'œil ciblé. Cela conduit la plupart du temps à une mauvaise délivrance du collyre selon la posologie prescrite : trop de collyre appliqué à l'œil ciblé ou la goute de collyre tombe à côté de l'œil cible, et ce, sans parler du désagrément pour le patient pour réaliser le geste, même si ce dernier fait appel à une tierce personne pour ce faire. Il n'existe pas sur le marché à ce jour de dispositif de délivrance d'une goutte de collyre qui permette au patient de mettre une goutte de collyre dans les yeux sans pencher la tête en arrière. Des dispositifs d'aide à la délivrance existent mais leurs conceptions requièrent également que le patient penche la tête en arrière pour mettre une goutte de collyre dans les yeux quand il les utilise en association avec les dispositifs de délivrance d'une goutte de collyre existants.

Afin de palier une partie de ces inconvénients, le document US 6 869 421 décrit un dispositif de délivrance d'une goutte de collyre pour une présentation non gravitationnelle de la goutte de collyre. Pour cela, le dispositif de délivrance de ce document présente une surface de présentation au fond de laquelle débouche une série de tubes capillaires permettant d'amener le collyre depuis le flacon et de former la goutte au niveau de la surface de présentation et de retenir celle-ci en place jusqu'à une inclinaison prédéterminée de délivrance à partir de laquelle la goutte est déposée ou tombe sur la surface de l'œil ciblé du patient. La délivrance se fait en inclinant la tête du patient. Toutefois, le geste reste délicat pour le patient.

Un dispositif de délivrance d'une goutte de collyre comprenant une tête de délivrance, un conduit d'amenée du collyre et une surface de délivrance au voisinage de l'extrémité de distribution est connu du document US 2005/288640.

### EXPOSE DE L'INVENTION

Un but de l'invention est de fournir un dispositif de délivrance d'une goutte de collyre qui ne présente pas les inconvénients précités, en particulier qui permette la délivrance d'une goutte de collyre selon le dosage prescrit et ce sans que le patient ait à incliner la tête en arrière et lever le bras au-dessus de la tête.

A cette fin, il est prévu, selon l'invention, un dispositif de délivrance d'une goutte de collyre, comportant ou non un ou plusieurs principes actifs, comprenant une tête de délivrance comportant un conduit d'amenée du collyre, le conduit d'amenée ayant une extrémité de distribution débouchante, et une surface de délivrance au voisinage de l'extrémité de distribution, la surface de délivrance s'étendant sur au moins un côté et en dessous de l'extrémité de distribution, le dispositif étant en position verticale, la tête de délivrance en haut, l'extrémité de distribution étant agencée de sorte à générer la goutte de collyre vers la surface de délivrance, et ainsi à permettre au patient de mettre la goutte de collyre dans un œil sans avoir à incliner la tête en arrière.

Avantageusement, mais facultativement, le dispositif de délivrance d'une goutte de collyre selon l'invention présente au moins l'une des caractéristiques techniques suivantes :
- l'extrémité de distribution est asymétrique par rapport à un axe longitudinal du dispositif ;
- l'extrémité de distribution présente une ouverture inclinée vers la surface de délivrance ;
- l'ouverture inclinée comporte une surface inclinée ;
- le conduit d'amenée comporte une portion distale inclinée par rapport à un axe longitudinal du dispositif, l'extrémité de distribution étant positionnée sur la portion distale inclinée ;
- le conduit d'amenée comprend une surface tampon d'accroche située à l'extrémité proximale agencée de sorte à permettre une rétention d'une goutte et de la calibrer ;
- le conduit d'amenée comporte des moyens formant pompe de distribution ;
- les moyens formant pompe de distribution comprennent une pompe de type péristaltique ;
- le conduit d'amenée comprend un filtre antibactérien ;
- le conduit d'amenée comprend une valve antiretour ;
- la surface de délivrance comprend un rebord de délivrance opposé à l'extrémité de distribution ;
- la surface de délivrance comporte un fond plat, en portion d'ovoïde ou en portion de sphère ; et,
- la surface de délivrance est hydrophobe.

### BREVE DESCRIPTION DES FIGURES

D'autres caractéristiques et avantages de l'invention ressortiront à la lecture de la description qui suit d'un mode de réalisation de l'invention. Aux dessins annexés :
[Fig.1] est une vue en coupe schématisée d'un premier mode de réalisation d'un dispositif de délivrance d'une goutte de collyre selon l'invention ;
[Fig.2] est une vue partielle en trois dimensions du dispositif de délivrance de la figure 1 ;
[Fig.3] est une vue partielle en coupe du dispositif de délivrance de la figure 1 ; [Fig.4a] et
[Fig.4b] sont des vues tridimensionnelles d'un deuxième mode de réalisation d'un dispositif de délivrance d'une goutte de collyre selon l'invention ;
[Fig.5] est une vue en trois dimensions d'une variante de réalisation du dispositif de délivrance de la figure 1 sous la forme d'un flacon unidose ;
[Fig.6] est une vue en trois dimensions d'une variante de réalisation du dispositif de délivrance des figures 4a et 4b ;
[Fig.7a] et
[Fig.7b] et
[Fig.7c] sont des vues partielles en coupe de trois variantes de réalisation du dispositif des figures 4a, 4b ou 6 ;
[Fig.8a] et
[Fig.8b] sont de vues partielles en coupe d'une quatrième variante de réalisation du dispositif de délivrance des figures 4a et 4b ou 6 ;
[Fig.9a] est une vue partielle en coupe d'un troisième mode de réalisation d'un dispositif de délivrance d'une goutte de collyre selon l'invention ;
[Fig.9b] et
[Fig.9c] sont des vues de détail en coupe de la tête du dispositif de délivrance de la figure 9a ;
[Fig.10] est une vue partielle en coupe d'une réalisation effective du dispositif de la figure 7c ;
[Fig.11] est une vue partielle en coupe d'une réalisation pratique du dispositif de la figure 7a ; et,
[Fig.12] est une vue partielle en coupe d'une autre variante de réalisation du dispositif de délivrance d'une goutte de collyre selon l'invention.

Pour plus de clarté, les éléments identiques ou similaires sont repérés par des signes de référence identiques sur l'ensemble des figures.

### DESCRIPTION DETAILLEE D'UN MODE DE REALISATION

En remarque liminaire, nous entendons dans l'ensemble de la description par « dispositif de délivrance d'une goutte de collyre » tout type de dispositif ménagé à cette fin : les flacons mono ou multidose, les têtes ou bouchons destinés à coopérer avec des flacons indépendants, etc...

D'autre part, la description qui va suivre des différents éléments et caractéristiques de modes de réalisation d'un dispositif de délivrance d'une goutte de collyre selon l'invention est réalisée avec ledit dispositif en position debout verticale : c'est-à-dire ledit dispositif en position verticale, la tête de délivrance étant en haut, comme cela est illustré dans les figures, et ainsi permettre au patient de mettre une goutte de collyre dans un œil sans incliner la tête en arrière.

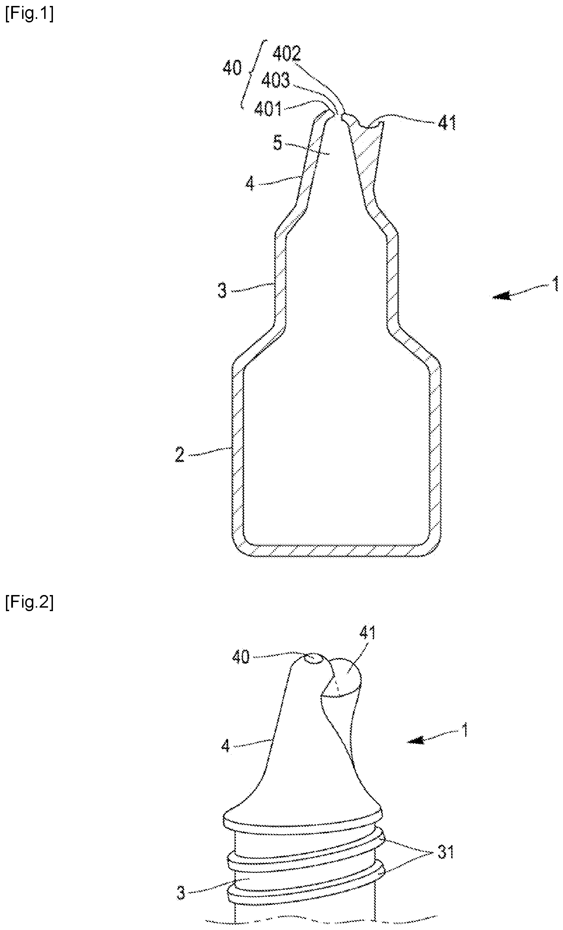

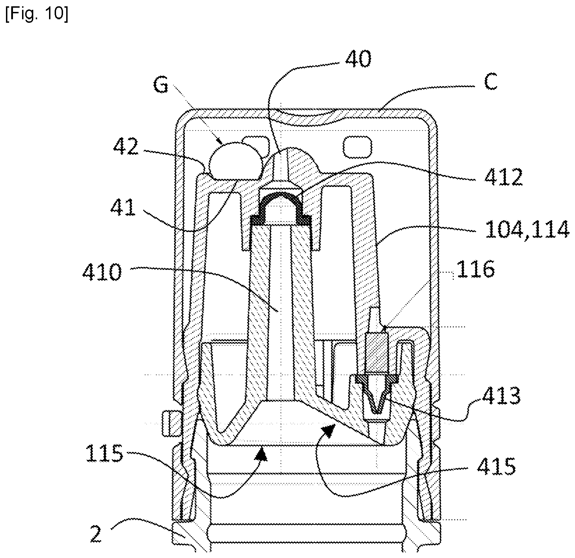

En référence aux figures 1 à 3, nous allons décrire un premier mode de réalisation d'un dispositif de délivrance d'une goutte de collyre 1 selon l'invention. Le dispositif de délivrance d'une goutte de collyre 1 selon l'invention comporte, ici, en position verticale, une tête de délivrance étant en haut, un flacon 2 souple, un support de tête 3 et une tête de délivrance 4. Le flacon 2, le support de tête 3 et la tête 4 étant un ensemble monobloc venu de matière les uns avec les autres. Le support de tête comporte, ici, un filetage permettant de coopérer avec un filetage complémentaire d'un bouchon de protection de la tête 4. La tête 4 comporte à son sommet, selon les figures, une extrémité de distribution 40 débouchante associée à une surface de délivrance 41. La surface de délivrance 41 est positionnée de manière adjacente à l'extrémité de distribution 40 de sorte à ce que la surface de délivrance 41 s'étende sur le côté ou latéralement par rapport à l'extrémité de distribution 40. D'autre part, la surface de délivrance 41 s'étend en-dessous de l'extrémité de distribution 40, en regard des figures (c'est-à-dire le dispositif de délivrance d'une goutte de collyre selon l'invention étant en position verticale, une tête de délivrance étant en haut). Autrement dit, l'extrémité de distribution 40 s'étend au-dessus, ou en saillie, de la surface de délivrance 41. De préférence, la surface de délivrance 41 est de forme sensiblement concave et présente un rebord de délivrance 42 (voir sur les figures 10 à 12), extérieur, opposé à l'extrémité de distribution 40. En variante de réalisation, la surface de délivrance 41 est rapportée sur la tête de délivrance 4, notamment comme une pièce indépendante de ladite tête de délivrance.

La tête de délivrance 4 comprend un conduit d'amenée 5 dont une extrémité distale est l'extrémité de distribution 40. A l'opposée, une extrémité proximale du conduit d'amenée 5 débouche dans le flacon 2 et est de forme évasée. L'extrémité de distribution 40 comporte une ouverture 403 délimitée par une bordure 401,402. Cette bordure est, ici, globalement inclinée vers la surface de délivrance 41, la bordure présentant un bord haut 401 et un bord bas 402 diamétralement opposé. Le bord bas 402 est adjacent à la surface de délivrance 41. Ainsi, l'ouverture 403 est inclinée vers la surface de délivrance 41. Au surplus, cela donne une forme asymétrique à l'extrémité de distribution 40 par rapport à une axe longitudinal du dispositif de délivrance d'une goutte de collyre 1 selon l'invention.

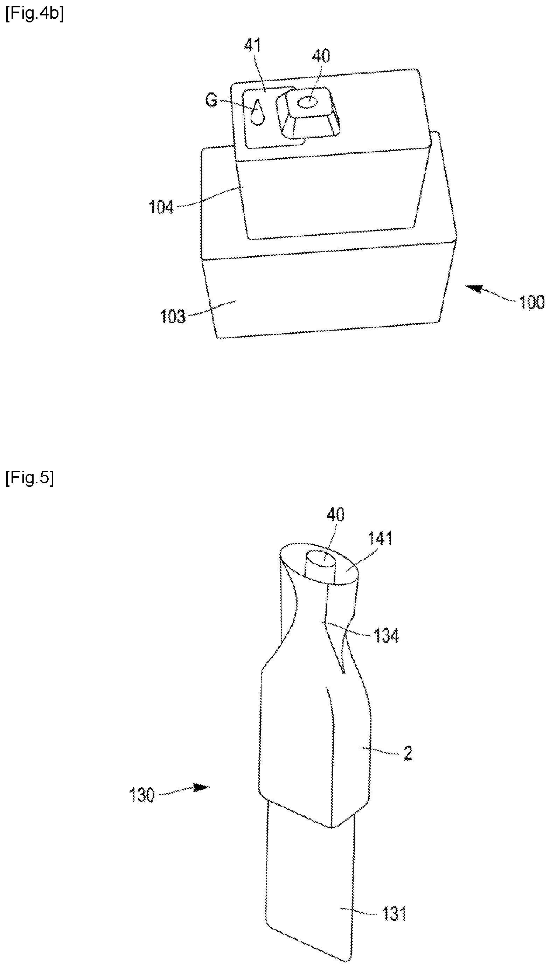

En référence à la figure 5, nous allons décrire une variante de réalisation du dispositif de délivrance d'une goutte de collyre 1 selon l'invention précédemment décrit. Le dispositif de délivrance d'une goutte de collyre 1 selon l'invention est une version multidose alors que la variante de réalisation du dispositif de délivrance d'une goutte de collyre 130 selon l'invention de la figure 5 est une version monodose ou à dose unique de collyre. Globalement, le dispositif de délivrance d'une goutte de collyre 130 selon l'invention est monobloc et comporte, comme précédemment, en position verticale, une tête de délivrance étant en haut, un flacon 2 souple surmonté d'une tête de délivrance 134. La tête de délivrance 134 comprend, en un sommet, une extrémité de distribution 40, qui correspond à l'extrémité distale du conduit d'amenée, et s'étendant au-dessus d'une surface de délivrance 141 qui entoure ladite extrémité de distribution 40. Le conduit d'amenée de la tête de délivrance 134 est, dans sa partie proximale, de forme évasée et fait la jonction avec le flacon 2. Le dispositif de délivrance d'une goutte de collyre 130 selon l'invention comporte en outre une languette de manipulation 131, connue en soi, située sous le flacon 2.

Nous allons maintenant décrire brièvement une utilisation des dispositifs de délivrance d'une goutte de collyre 1 et 130 selon l'invention précédemment décrits. Dans un premier temps, l'utilisateur renverse le dispositif de délivrance d'une goutte de collyre 1,130 selon l'invention pour permettre au collyre d'entrer en contact avec la forme évasée à l'extrémité proximale du conduit d'amenée 5, cette forme évasée formant une surface tampon d'accroche pour une quantité prédéterminée de collyre. Le dispositif de délivrance d'une goutte de collyre 1,130 selon l'invention est alors à nouveau placé en position verticale, et il reste alors attaché à la surface tampon d'accroche de l'extrémité proximale du conduit d'amenée 5 une goutte de volume fini et calibrée. L'utilisateur appuie alors sur les parois du flacon 2 souple jusqu'à faire perler la goutte de collyre au niveau de l'extrémité de distribution 40, goutte de collyre qui ensuite glisse sur la surface de délivrance 41, 141 où elle reste. Cette opération s'effectue avec le dispositif de délivrance d'une goutte de collyre 1,131 selon l'invention en position verticale, la tête de délivrance 4,134 étant en haut. En gardant cette position verticale du dispositif de délivrance d'une goutte de collyre 1,131 selon l'invention, l'utilisateur, gardant la tête droite, approche ce dernier de la joue située sous l'œil ciblé à traiter en positionnant le rebord de délivrance de la surface de délivrance 41,141 contre le bord de la paupière inférieure de l'œil ciblé. Une fois le rebord extérieur de la surface de délivrance 41,141 au contact du bord de la paupière inférieure, la goutte de collyre présente sur la surface de délivrance 41,141 « glisse » dans le cul-de-sac conjonctival de l'œil ciblé par capillarité : la goutte de collyre est comme aspirée par le fluide présent dans le cul-de-sac conjonctival.

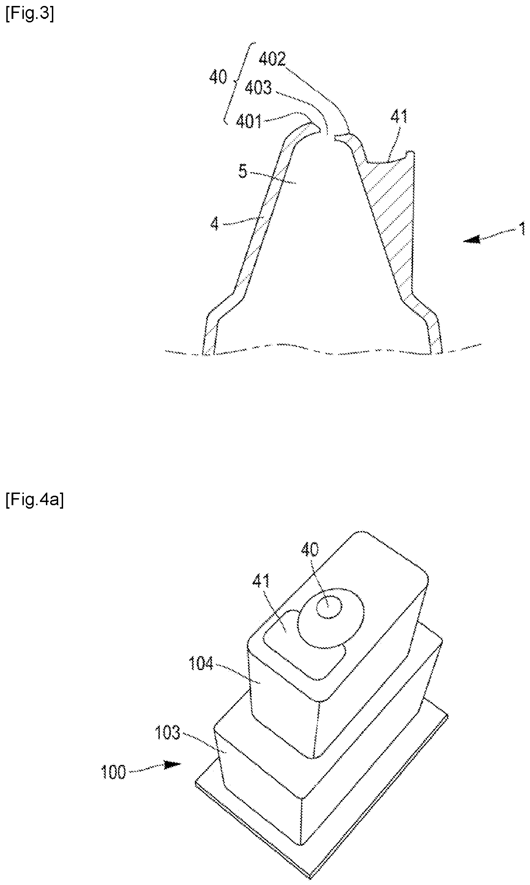

Maintenant, en référence aux figures 4a et 4b nous allons décrire un deuxième mode de réalisation d'un dispositif de délivrance d'une goutte de collyre 100 selon l'invention. Il est à noter que la figure 4b représente le dispositif de délivrance d'une goutte de collyre 100 selon l'invention de la figure 4a avec une goutte de collyre présente sur la surface de délivrance 41. Le dispositif de délivrance d'une goutte de collyre 100 selon l'invention est de section globalement rectangulaire et est sous la forme, ici, d'un bouchon destiné à venir se fixer sur un flacon contenant du collyre qui peut être souple ou rigide. Ainsi, le dispositif de délivrance d'une goutte de collyre 100 selon l'invention comporte en position verticale, une tête de délivrance étant en haut, un support de tête 103 de section rectangulaire, surmontée d'une tête de délivrance 104, elle-même de section rectangulaire, plus petite que celle du support de tête 103. Au sommet de la tête de délivrance 104, le dispositif de délivrance d'une goutte de collyre 100 selon l'invention comporte une surface de délivrance 41 adjacente d'une extrémité de distribution 40 qui s'étend en saillie au-dessus de ladite surface de délivrance 41. Comme précédemment, l'extrémité de distribution 40 comporte une ouverture inclinée vers la surface de délivrance 41. De plus, l'ouverture inclinée comporte une surface inclinée, de forme sensiblement concave au fond de laquelle débouche le conduit d'amenée 410.

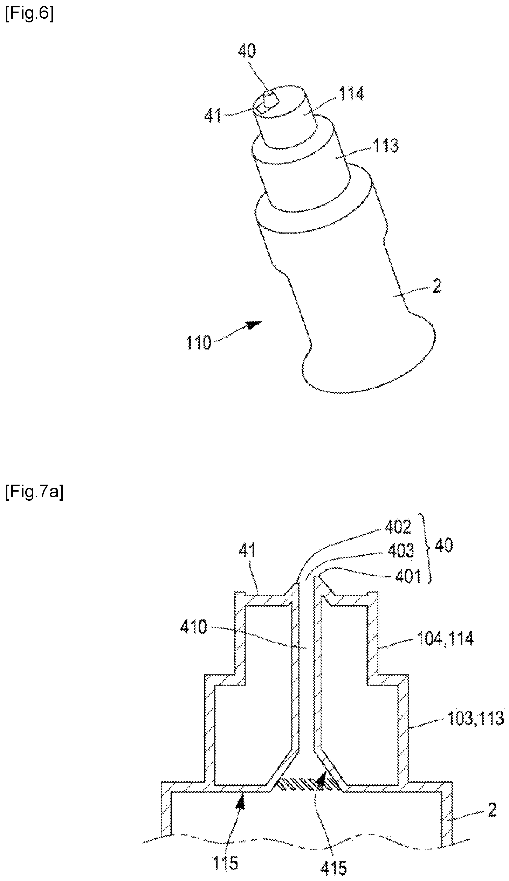

En variante de réalisation, la section globale du dispositif de délivrance d'une goutte de collyre 100 selon l'invention est de forme circulaire ou ovale. La forme ovale est illustrée à la figure 6, où le dispositif de délivrance d'une goutte de collyre 110 selon l'invention comporte un support de tête 113 et une tête de délivrance 114 de section ovale.

En référence à la figure 7a, nous allons décrire plus en détail le dispositif de délivrance d'une goutte de collyre 100 ou 110 selon l'invention des figures précédentes 4a, 4b et 6. La tête de délivrance 104,114 comporte un conduit d'amenée 410 dont l'extrémité distale comporte l'extrémité de distribution 40. A l'opposé, l'extrémité proximale du conduit d'amenée débouche dans le flacon 2 au niveau d'une surface 115.

L'extrémité de distribution 40, ici, sensiblement en forme de cône, comporte une ouverture 403 délimitée par une bordure 401,402, comme cela a été déjà décrit pour le premier mode de réalisation du dispositif de délivrance d'une goutte de collyre 1 selon l'invention. De nouveau, cette bordure est, ici, globalement inclinée vers la surface de délivrance 41, la bordure présentant un bord haut 401 et un bord bas 402 diamétralement opposé. Le bord bas 402 est adjacent à la surface de délivrance 41. Ainsi, l'ouverture 403 est inclinée vers la surface de délivrance 41. Au surplus, cela donne une forme asymétrique à l'extrémité de distribution 40 par rapport à un axe longitudinal du dispositif de délivrance d'une goutte de collyre 1 selon l'invention.

Quant à elle, l'extrémité proximale comporte une surface tampon d'accroche 415 de forme concave dans le fond de laquelle débouche le conduit d'amenée 410. Ici, la surface tampon d'accroche 415 est, ici, de forme tronconique. De manière générale, la surface tampon d'accroche 415 est de forme concave adaptée : de forme évasée, portion d'ovoïde, de prisme, etc... Cette surface tampon d'accroche 415 est conformée et dimensionnée de sorte à retenir, par tension de surface, une quantité prédéfinie de collyre qui correspond à la goutte de collyre qui doit être distribuée dans l'œil d'un patient par le dispositif de délivrance d'une goutte de collyre 100,110 selon l'invention. D'autre part, un revêtement de surface de la surface tampon d'accroche 415 peut être aussi prévu à cet effet : cette dernière peut présenter un état de surface ayant une certaine rugosité adaptée, être recouverte d'une grille, ou encore d'une couche de matériau hydrophile compatible avec un usage médical qui permette de faciliter la rétention de la goutte de collyre dans un volume délimité par la surface tampon d'accroche 415.

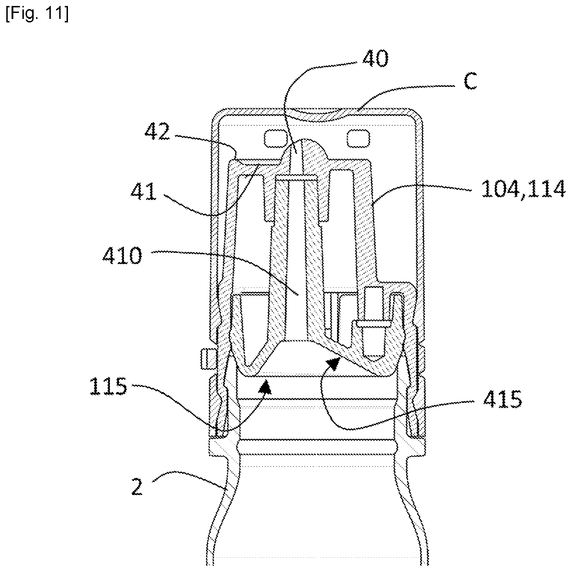

En figure 11, est illustrée une réalisation effective du dispositif de délivrance d'une goutte de collyre selon l'invention qui vient d'être décrit en référence à la figure 7a. Sur cette figure 11, un capuchon de protection C a été représenté.

Comme indiqué précédemment, la surface de délivrance 41 est de forme sensiblement concave et présente un rebord de délivrance 42, extérieur, opposé à l'extrémité de distribution 40. La surface de délivrance 41 peut présenter un fond plat comme illustré, ou encore est de forme arrondie en portion d'ovoïde ou de sphère.

Lors d'une utilisation, le patient ou l'utilisateur du dispositif de délivrance d'une goutte de collyre 100,110 selon l'invention met en position tête-bêche ledit dispositif de délivrance d'une goutte de collyre 100,110 selon l'invention : la tête de délivrance 104,114 étant alors en bas, le collyre contenu dans le flacon 2 recouvre la surface 115 et la surface tampon d'accroche 415 est recouverte par ledit collyre. Ensuite, l'utilisateur ou le patient remet en position verticale le dispositif de délivrance d'une goutte de collyre 100,110 selon l'invention, la tête de délivrance 104,114 positionnée en haut. Dans cette position, le collyre retombe dans le fond du flacon et une quantité de collyre prédéterminée est retenue par la surface tampon d'accroche 415. Dès lors, un volume d'air est dans le flacon positionné entre le collyre restant et ladite quantité de collyre prédéterminée retenue par la surface tampon d'accroche 415. Ensuite, l'utilisateur ou le patient appuie sur le flacon 2, la pression qui s'installe dans le flacon 2 pousse alors la quantité de collyre prédéterminée depuis la surface tampon d'accroche 415 dans le conduit d'amenée 410 vers l'extrémité de distribution 40 où la goutte de collyre G va alors perler puis glisser sur la surface de délivrance 41. En gardant la position verticale du dispositif de délivrance d'une goutte de collyre 100,110 selon l'invention, la tête de délivrance étant en haut, l'utilisateur, gardant la tête droite, approche ce dernier de la joue située sous l'œil ciblé à traiter en positionnant le rebord de délivrance 42 de la surface de délivrance 41 contre le bord de la paupière inférieure de l'œil ciblé. Une fois le rebord extérieur de la surface de délivrance 41 au contact du bord de la paupière inférieure, la goutte de collyre G présente sur la surface de délivrance 41 « glisse » dans le cul-de-sac conjonctival de l'œil ciblé par capillarité : la goutte de collyre G est comme aspirée par le fluide présent dans le cul-de-sac conjonctival.

En variante de réalisation, il peut être prévu que le flacon 2 soit rigide avec une surface délimitée souple de sorte à contrôler facilement une éjection de la goutte de collyre depuis la surface tampon d'accroche 415 jusqu'à son glissement depuis l'extrémité de distribution 40, via le conduit d'amenée 410.

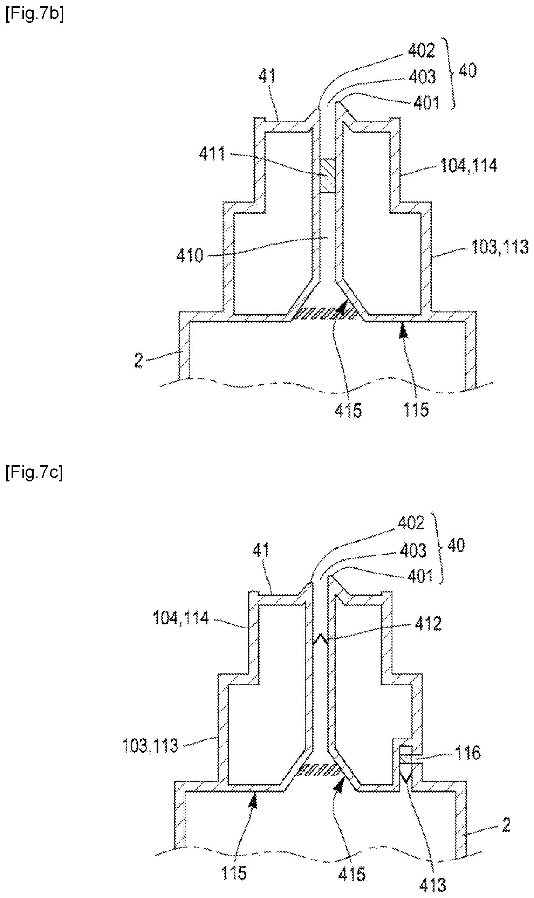

Dans une première variante de réalisation illustrée à la figure 7b, le conduit d'amenée 410 comporte un filtre bactéricide ou antibactérien 411.

Dans une deuxième variante de réalisation illustrée à la figure 7c, le conduit d'amenée comporte une valve anti-retour 412. Au surplus, le support de tête 103,113 comporte un conduit 116 d'entrée d'air dans le flacon 2. Ce conduit 116 d'entrée d'air est équipé d'une valve anti-retour 413 en amont de laquelle se trouve un filtre bactéricide ou antibactérien.

La figure 10 illustre une réalisation effective de cette deuxième variante de réalisation du dispositif de délivrance d'une goutte de collyre selon l'invention.

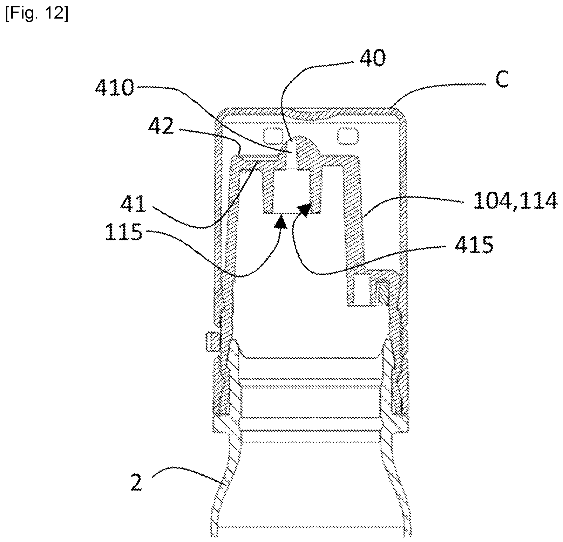

En référence à la figure 12, nous allons décrire une autre variante de réalisation d'un dispositif de délivrance d'une goutte de collyre selon l'invention. Cette variante se différencie des modes de réalisations précédemment décrit par le fait que le tube d'amenée 410 est très court. En effet, une longueur de ce tube d'amenée 410 est de l'ordre d'une épaisseur de la tête de délivrance au niveau de l'extrémité de distribution 40, la surface 115 et la surface tampon d'accroche 415 étant positionnée au plus proche de ladite extrémité de distribution 40. Cela permet d'obtenir une tête de délivrance qui soit optimisée en volume de matériau et en nombre de composants utilisés pour sa fabrication.

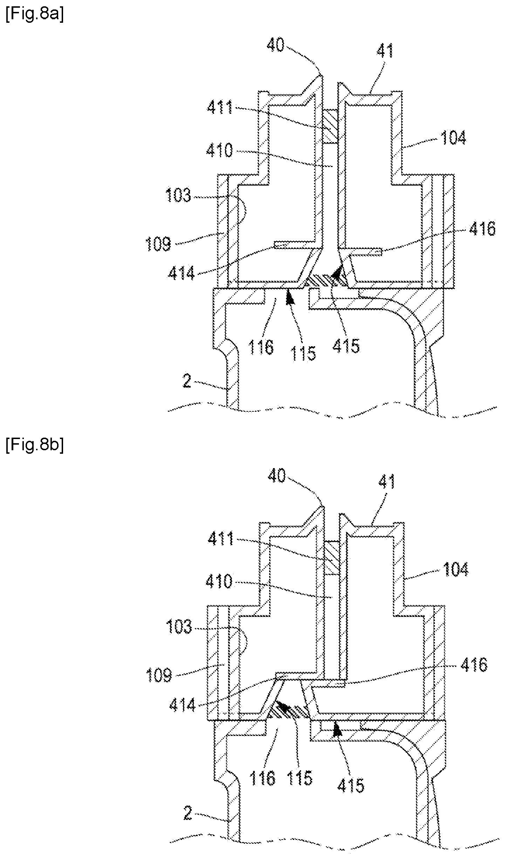

Dans une troisième variante illustrée aux figures 8a et 8b, le dispositif de délivrance d'une goutte de collyre 100,110 selon l'invention comporte, au niveau du support de tête 103 un tiroir 109 coulissant perpendiculairement à l'axe longitudinal du dispositif de délivrance d'une goutte de collyre 100,110 selon l'invention entre une position ouverte de délivrance illustrée en figure 8a et une position fermée illustrée en figure 8b. En variante de réalisation, le tiroir 109 pivote autour d'un axe de rotation parallèle à un axe longitudinal du dispositif de distribution d'une goutte de collyre 100,110 selon l'invention. Le tiroir 109 comporte la surface 115 ainsi que la surface tampon d'accroche 415 muni d'un flasque 416. Le conduit d'amenée comportant un flasque 414 symétrique. En position ouverte de délivrance, le volume délimité par la surface tampon d'accroche 415 est en connexion fluidique avec le conduit d'amenée 410 pour une éjection vers la surface de délivrance 41 d'une goutte de collyre. La surface tampon d'accroche 415 est coupé du flacon 2. En position fermée du tiroir 109, la surface tampon d'accroche 415 est déplacée de sorte à être isolée du conduit d'amenée 410 et à être en connexion fluidique avec le flacon 2. Dans cette position le flasque 414 du conduit d'amenée bouche le haut de la surface tampon d'accroche 415 et le flasque 416 obture le conduit d'amenée 410. Dans cette position fermée, en retournant tête-bêche le dispositif de délivrance d'une goutte de collyre 100,110 selon l'invention, le volume délimité par la surface tampon d'accroche 415 est rempli par une quantité prédéterminée de collyre. L'utilisation est par ailleurs, *mutatis mutandis*, la même.

En variante de réalisation (non illustrée) de tous les modes précédemment décrits, le conduit d'amenée comporte une portion distale inclinée par rapport à un axe longitudinal du dispositif, l'extrémité de distribution étant positionnée sur la portion distale inclinée.

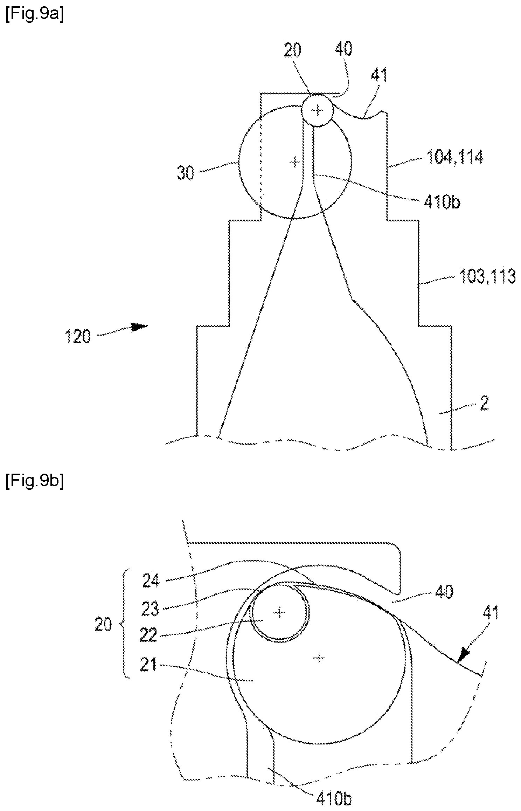

En référence maintenant aux figures 9a à 9c, nous allons décrire un troisième mode de réalisation d'un dispositif de délivrance d'une goutte de collyre 120 selon l'invention. Ce mode de réalisation se différencie avec le deuxième mode de réalisation précédemment décrit par la présence de moyens formant pompe de distribution 20,30. Ce dispositif de délivrance d'une goutte de collyre 120 selon l'invention comporte un flacon 2 comprenant un sac réservoir de collyre directement connecté fluidiquement avec les moyens formant pompe 20,30 via un conduit d'amenée 410b souple.

Au niveau de la tête de distribution 104,114, le dispositif de délivrance d'une goutte de collyre 120 selon l'invention comporte une molette de distribution 30 permettant de mettre en œuvre un galet 21. En variante de réalisation, la molette 30 peut être positionnée au niveau du support de tête 103,113 du dispositif de délivrance d'une goutte de collyre 120 selon l'invention. Le galet 21 comporte un excentrique 22 qui vient écraser un tube flexible 23 prolongeant le conduit d'amenée vers l'extrémité de distribution 40 surplombant la surface de délivrance 41. Le galet 21, entrainé en rotation par la molette 30 est monté mobile à rotation selon son axe dans un logement cylindrique de révolution 25. De plus, le galet 21 comporte une sorte de méplat 24 en amont de l'excentrique 22, le galet 21 tournant dans le sens des aiguilles d'une montre sur les figures 9a à 9c. L'ensemble fonctionne comme une pompe péristaltique.

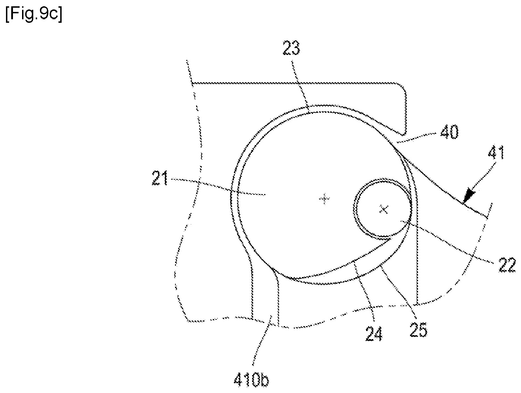

La présence du méplat 24 permet de doser la quantité de collyre que la pompe va éjecter à travers l'extrémité de distribution 40 vers la surface de délivrance 41. En effet en position fermée de non-utilisation, illustrée à la figure 9c, le galet 21 écrase complètement le tube flexible 23, isolant le conduit d'amenée 410b de l'ouverture de l'extrémité de distribution 40. En position de fonctionnement, comme en partie illustrée à la figure 9b, l'excentrique 22 pousse la quantité de collyre défini par le méplat 24 le long du conduit flexible 23 vers l'extrémité de distribution 40.

En utilisation, une fois que l'utilisateur a généré la goutte de collyre à administrer sur la surface de délivrance 41 via la molette 30, le mode opératoire est similaire à ce qui a déjà été décrit précédemment pour les modes de réalisation du dispositif de délivrance d'une goutte de collyre selon l'invention. Une telle structure d'un dispositif de délivrance d'une goutte de collyre telle qui vient d'être décrite, et illustrée par les figures 1 à 12, permet d'assurer des posologies de délivrance d'une seule et unique goutte de collyre. Les dispositifs existants de ce type ne permettent pas une telle sécurité dans la délivrance d'une posologie d'une goutte unique.

Dans tous les modes de réalisation, la surface de délivrance 41,141 est de forme concave, comme une cuillère, ou plane et est délimitée à l'opposé de l'extrémité de distribution 40 par un rebord 42 de délivrance. De manière générale, la surface de délivrance 41,141 est conformée de sorte à faciliter un transfert par capillarité de la goutte de collyre, qui est présente sur ladite surface de délivrance 41,141, dans le cul-de-sac conjonctival de l'œil ciblé tout en ne laissant pas de résidus aqueux sur la surface de délivrance 41,141. A cette fin, la surface de délivrance 41,141 peut être recouverte ou réalisée dans un matériau hydrophobe.

Bien entendu, il est possible d'apporter à l'invention de nombreuses modifications sans pour autant sortir du cadre de celle-ci.

## Revendications

1. Dispositif de délivrance d'une goutte de collyre (1;100;110;120;130), comportant ou non un ou plusieurs principes actifs, comprenant une tête de délivrance (4;104;114 ;134) comportant un conduit d'amenée (5;410;410b) du collyre, le conduit d'amenée ayant une extrémité de distribution (40) débouchante, et une surface de délivrance (41;141) au voisinage de l'extrémité de distribution, **caractérisé en ce que**, le dispositif étant en position verticale, la tête de délivrance en haut, la surface de délivrance s'étend sur au moins un côté et en dessous de l'extrémité de distribution, l'extrémité de distribution étant agencée de sorte à générer la goutte de collyre (G) vers la surface de délivrance.

2. Dispositif selon la revendication 1, **caractérisé en ce que** l'extrémité de distribution est asymétrique par rapport à un axe longitudinal du dispositif.

3. Dispositif selon la revendication 2, **caractérisé en ce que** l'extrémité de distribution présente une ouverture inclinée vers la surface de délivrance.

4. Dispositif selon la revendication 3, **caractérisé en ce que** l'ouverture inclinée comporte une surface inclinée.

5. Dispositif selon la revendication 2, **caractérisé en ce que** le conduit d'amenée comporte une portion distale inclinée par rapport à un axe longitudinal du dispositif, l'extrémité de distribution étant positionnée sur la portion distale inclinée.

6. Dispositif selon l'une des revendications 1 à 5, **caractérisé en ce que** le conduit d'amenée comprend une surface tampon d'accroche (415) située à l'extrémité proximale agencée de sorte à permettre une rétention d'une goutte et de la calibrer.

7. Dispositif selon l'une des revendications 1 à 6, **caractérisé en ce que** le conduit d'amenée comporte des moyens formant pompe de distribution (20;30).

8. Dispositif selon la revendication 7, **caractérisé en ce que** les moyens formant pompe de distribution comprennent une pompe de type péristaltique.

9. Dispositif selon l'une des revendications 1 à 8, **caractérisé en ce que** le conduit d'amenée comprend une valve antiretour (412).

10. Dispositif selon l'une des revendications 1 à 9, **caractérisé en ce que** le conduit d'amenée comprend un filtre antibactérien et/ou bactéricide (411).

11. Dispositif selon l'une des revendications 1 à 10, **caractérisé en ce que** la surface de délivrance comprend un rebord de délivrance (42) opposé à l'extrémité de distribution.

12. Dispositif selon la revendication 11, **caractérisé en ce que** la surface de délivrance comporte un fond plat, en portion d'ovoïde ou en portion de sphère.

13. Dispositif selon l'une des revendications 1 à 12, **caractérisé en ce que** la surface de délivrance est hydrophobe.

## Patentansprüche

1. Vorrichtung zum Abgeben eines Tropfens Augentropfen (l; 100; 110; 120; 130), der einen oder mehrere Wirkstoffe aufweist oder nicht, umfassend einen Abgabekopf (4;104;114;134), der einen Zuführkanal (5;410;410b) für die Augentropfen aufweist, wobei der Zuführkanal ein durchgehendes Abgabeende (40), und eine Ausgabefläche (41;141) in der Nähe des Abgabeendes hat, **dadurch gekennzeichnet, dass** die Vorrichtung in vertikaler Position ist, mit dem Abgabekopf nach oben, die Ausgabefläche sich auf mindestens einer Seite und unterhalb des Abgabeendes erstreckt, wobei das Abgabeende so angeordnet ist, dass es den Tropfen Augentropfen (G) zu der Ausgabefläche hin erzeugt.

2. Vorrichtung nach Anspruch 1, **dadurch gekennzeichnet, dass** das Abgabeende in Bezug auf eine Längsachse der Vorrichtung asymmetrisch ist.

3. Vorrichtung nach Anspruch 2, **dadurch gekennzeichnet, dass** das Abgabeende eine zu der Ausgabefläche geneigte Öffnung besitzt.

4. Vorrichtung nach Anspruch 3, **dadurch gekennzeichnet, dass** die geneigte Öffnung eine geneigte Oberfläche aufweist.

5. Vorrichtung nach Anspruch 2, **dadurch gekennzeichnet, dass** der Zuführkanal einen gegenüber einer Längsachse der Vorrichtung geneigten distalen Abschnitt aufweist, wobei das Abgabeende auf dem geneigten distalen Abschnitt positioniert ist.

6. Vorrichtung nach einem der Ansprüche 1 bis 5, **dadurch gekennzeichnet, dass** der Zuführkanal eine an dem proximalen Ende angeordnete Anschlagpufferfläche (415) umfasst, die so angeordnet ist, dass sie ein Zurückhalten eines Tropfens ermöglicht und ihn kalibriert.

7. Vorrichtung nach einem der Ansprüche 1 bis 6, **dadurch gekennzeichnet, dass** der Zuführkanal Mittel aufweist, die eine Verteilpumpe (20;30) bilden.

8. Vorrichtung nach Anspruch 7, **dadurch gekennzeichnet, dass** die Mittel, die eine Verteilpumpe bilden, eine Pumpe des peristaltischen Typs umfassen.

9. Vorrichtung nach einem der Ansprüche 1 bis 8, **dadurch gekennzeichnet, dass** der Zuführkanal ein Rückschlagventil (412) umfasst.

10. Vorrichtung nach einem der Ansprüche 1 bis 9, **dadurch gekennzeichnet, dass** der Zuführkanal einen antibakteriellen und/oder bakteriziden Filter (411) umfasst.

11. Vorrichtung nach einem der Ansprüche 1 bis 10, **dadurch gekennzeichnet, dass** die Ausgabefläche einen dem Abgabeende gegenüberliegenden Abgaberand (42) umfasst.

12. Vorrichtung nach Anspruch 11, **dadurch gekennzeichnet, dass** die Ausgabefläche einen flachen Boden, einen eiförmigen Abschnitt oder einen kugelförmigen Abschnitt aufweist.

13. Vorrichtung nach einem der Ansprüche 1 bis 12, **dadurch gekennzeichnet, dass** die Ausgabefläche wasserabweisend ist.

## Claims

1. A device (1; 100; 110; 120; 130) for dispensing an eyedrop, optionally having one or more active principles, comprising a dispensing head (4; 104; 114; 134) having a supply conduit (5; 410; 410b) for the eyedrop solution, the supply conduit having an open delivery end (40), and a dispensing surface (41; 141) in proximity to the delivery end, **characterized in that**, with the device in a vertical position with the dispensing head upward, the dispensing surface extends on at least one side of and below the delivery end, the delivery end being arranged to generate the eyedrop (G) of eyedrop solution toward the dispensing surface.

2. The device as claimed in claim 1, **characterized in that** the delivery end is asymmetrical with respect to a longitudinal axis of the device.

3. The device as claimed in claim 2, **characterized in that** the delivery end has an opening inclined toward the dispensing surface.

4. The device as claimed in claim 3, **characterized in that** the inclined opening has an inclined surface.

5. The device as claimed in claim 2, **characterized in that** the supply conduit has a distal portion inclined with respect to a longitudinal axis of the device, the delivery end being positioned on the inclined distal portion.

6. The device as claimed in any one of claims 1 to 5, **characterized in that** the supply conduit comprises an attachment buffer surface (415) situated at the proximal end and arranged so as to permit retention of a drop and to calibrate the drop.

7. The device as claimed in any one of claims 1 to 6, **characterized in that** the supply conduit has means forming a delivery pump (20; 30).

8. The device as claimed in claim 7, **characterized in that** the means forming a delivery pump comprises a peristaltic pump.

9. The device as claimed in any one of claims 1 to 8, **characterized in that** the supply conduit comprises a non-return valve (412).

10. The device as claimed in any one of claims 1 to 9, **characterized in that** the supply conduit comprises an antibacterial and/or bactericidal filter (411).

11. The device as claimed in any one of claims 1 to 10, **characterized in that** the dispensing surface comprises a dispensing rim (42) opposite the delivery end.

12. The device as claimed in claim 11, **characterized in that** the dispensing surface has a flat bottom, in the form of an ovoid portion or a portion of a sphere.

13. The device as claimed in any one of claims 1 to 12, **characterized in that** the dispensing surface is hydrophobic.
